(19) Europäisches Patentamt

European Patent Office

Office européen des brevets

(11) **EP 1 043 585 A2**

(12) **EUROPEAN PATENT APPLICATION**

(43) Date of publication:
**11.10.2000 Bulletin 2000/41**

(51) Int. Cl.$^7$: **G01N 33/18**, G01N 33/00, G01N 27/40

(21) Application number: **00107074.7**

(22) Date of filing: **05.04.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **05.04.1999 JP 9742099**

(71) Applicant: **Katayama, Hiroyuki
Chikushino-shi, Fukuoka-ken (JP)**

(72) Inventor: **Katayama, Hiroyuki
Chikushino-shi, Fukuoka-ken (JP)**

(74) Representative:
**Müller-Boré & Partner
Patentanwälte
Grafinger Strasse 2
81671 München (DE)**

(54) **Method and system for measuring partial pressure of material dissolved in liquid**

(57)     A method for measuring partial pressure of a material in a liquid includes a step of forming a gas-liquid equilibrium state between the liquid in which the material is dissolved and a carrier gas, and a step of measuring the partial pressure of the material dissolved in the liquid by utilizing vaporized material in the carrier gas which has reached the gas-liquid equilibrium state. The forming step includes a step of forming the gas-liquid equilibrium state between the liquid and the carrier gas present within a certain volume through a free liquid surface existing at each hole of a hydrophobic porous membrane including holes. The diameter and total area of the holes is defined such that the liquid cannot permeate through the hydrophobic porous membrane due to its surface tension but the material can permeate therethrough.

A system for measuring partial pressure of carbon dioxide dissolved in a liquid includes means for forming a gas-liquid equilibrium state of carbon dioxide between the liquid in which carbon dioxide is dissolved and a carrier gas, and means for measuring the partial pressure of carbon dioxide dissolved in the carrier gas which have reached the gas-liquid equilibrium state. The forming means includes a hydrophobic porous membrane having holes whose diameter and total area are defined, such that the liquid cannot permeate through the hydrophobic porous membrane due to its surface tension but carbon dioxide can permeate therethrough. The membrane includes an inner membrane surface contacting the carrier gas and an outer membrane surface contacting the liquid. The forming means further includes a carrier gas chamber open onto the inner membrane surface including an inlet opening through which the carder gas flows in and an outlet opening through which the carrier gas flows out. A gas-liquid equilibrium state is formed between the carrier gas inside the carrier gas chamber and the liquid through the free liquid surface existing at each hole.

FIG.1

**Description**

BACKGROUND OF THE INVENTION

Field of the Invention

The present invention relates to a method and a system for measuring partial pressure of a material dissolved in a liquid. More specifically, the present invention relates to a method and a system for measuring partial pressure of carbon dioxide dissolved in sea water.

Description of the Related Art

Recently, much attention has been paid to global warming as one of the global environmental problems. In order to prevent such global warming, reduction of the amount of discharged carbon dioxide has been internationally promoted.

However, of the total amount of carbon dioxide discharged into the atmosphere through the activities of human beings, only half accumulates in the atmosphere to become a major cause of global warming. Where and how much of the remaining amount of carbon dioxide is absorbed by forests and the sea has not been clarified yet. The solution of the so-called " missing sink " is considered to be essential for concretely determining the target amount of carbon dioxide to be reduced, and thus it is necessary to continuously monitor the partial pressure of carbon dioxide on the order of ppm level in sea water whose surface constantly changes.

Conventionally, a method for indirectly measuring the partial pressure of the carbon dioxide in sea water, a liquid phase, is known as a method for continuously measuring the partial pressure of carbon dioxide dissolved in sea water. According to this above method, an equilibrium state is formed between the gaseous phase and the liquid phase and the partial pressure of carbon dioxide is indirectly measured by utilizing the gaseous phase which have reached the equilibrium state. The equilibrium state between the gaseous phase and the liquid phase is defined to be a state in which the material movement between the gaseous phase(the carbon dioxide in this case) and the liquid phase through the liquid surface is apparently halted, and in particular, the equilibrium state formed after infinite time elapses is referred to as the natural equilibrium state.

A concentration gradient exists on either sides of the surface of contact between the gaseous and liquid phases, the free liquid surface, for instance. These gradients are called the liquid phase laminar film and the gaseous phase laminar film. In order to facilitate the material movement between the gaseous phase and the liquid phase, it is crucial to renew both films, especially the laminar film on the side of the liquid phase, whose dispersing speed is slow.

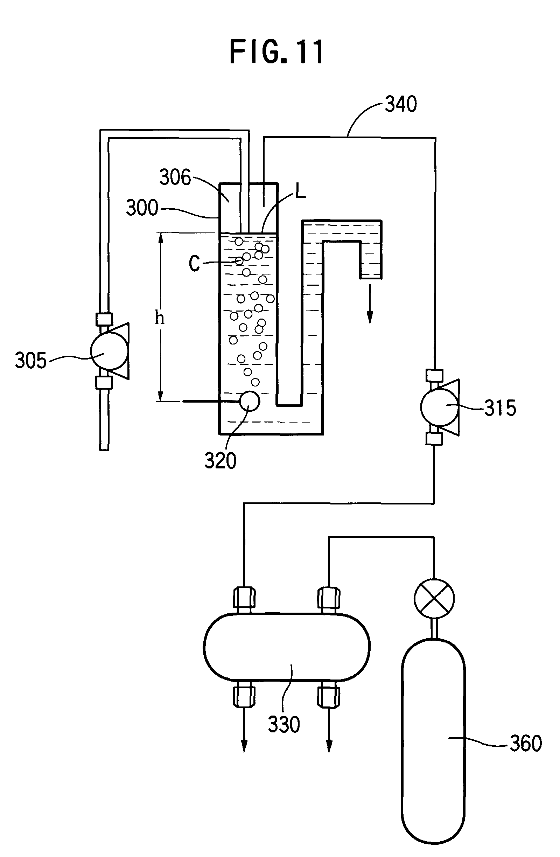

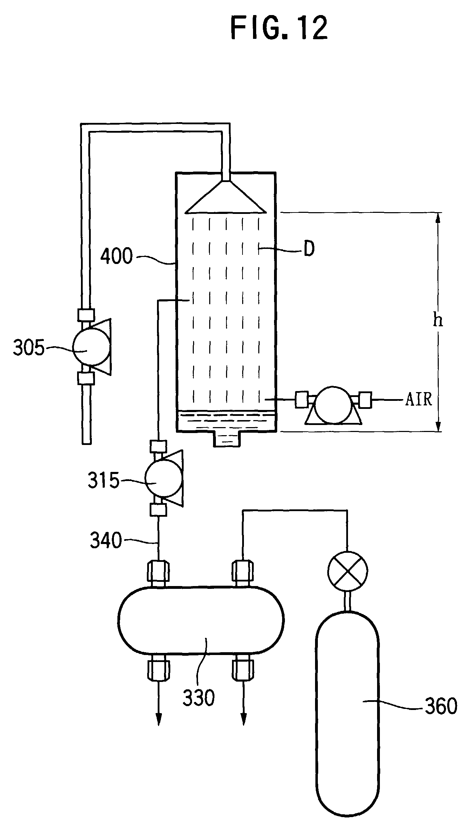

Conventional methods are divided into the bubble type measuring method and the shower type measuring method, which are differentiated from each other in how the equilibrium state is formed. Figures 11 and 12 show the bubble and the shower type systems, respectively. Each system includes means 100 for forming a carbon dioxide gas-liquid equilibrium state between the sea water in which the carbon dioxide is dissolved and a carrier gas (atmosphere), means 130 for measuring the partial pressure of carbon dioxide in the carrier gas which has reached the equilibrium state and a through-flow pipe 140 for connecting the forming means 100 and the measuring means 130 to supply the carrier gas to the forming means 100 and exhausting it from the means 130 to the atmosphere.

More specifically, with regard to the step of forming the equilibrium state by means 100 for forming the gas-liquid equilibrium state, as shown in Figure 11, the bubble type measuring method includes a step of forming the equilibrium state between the sea water outside the bubbles and the carrier gas inside the bubbles by utilizing bubbles. Namely, in this method, the carrier gas is blown into sampled sea water in a container 100 through a bubbler 120 by a pump 105 to form bubbles C of the carrier gas in the sea water. As a result, an equilibrium state is formed due to the fact that the bubbles C renew the laminar film on the side of the liquid phase while rising to the free liquid surface L (during the time period it takes them to rise by the distance h).

On the other hand, as shown in Figure 12, the shower type measuring method comprises a step of forming the equilibrium state by utilizing a shower ( droplets ). Sea water sampled by a pump 105 is showered into the carrier gas in the container 100, so that droplets D are formed in the carrier gas. Then, the equilibrium state is formed between the carrier gas outside the droplets and the sea water inside the droplets while a laminar film on the side of the gaseous phase is renewed. (During the time period it takes the droplets to fall the distance h)

As compared with the natural gas-liquid equilibrium state, both types enable the gas-liquid equilibrium state to be facilitated by forcibly contacting the gas with the liquid to renew the laminar film.

Next, as to the step of measuring the partial pressure of carbon dioxide utilizing the means 130 for measuring the partial pressure of carbon dioxide, the partial pressure of carbon dioxide inside the sample gas is measured by a Non-Dispersive Infrared Gas Analyzer (referred to as NDIR hereinafter) in both types. The NDIR 130 measures the partial pressure by detecting the intensity of light. It utilizes the fact that the absorbed wavelength of infrared rays differs depending on the gases. More specifically, it generally comprises a box cell through which a sample gas to be measured is passed, a light source for non-dispersive by directly light into the box cell, and a photodetector for detecting the intensity of the light absorbed by carbon dioxide in the sample gas. When sample gas from the container 100 is passed through the box cell via a through-flow pipe 140 while light is introduced from the light source into the box cell, the intensity of attenuated light is detected by the photodetector. The light entering the box is absorbed in proportion to the partial pressure of the carbon dioxide in the sample gas and thus attenuated. The partial pressure in the gas can therefore be indirectly measured by comparing the intensity of the attenuated light with that of light under the partial pressure of a reference gas 160. The sample gas is discharged after the measurement is completed.

In both measuring methods, the partial pressure of carbon dioxide in the sea water can be indirectly measured by measuring the partial pressure of carbon dioxide in the sample gas which has reached the gas-liquid equilibrium state.

In continuously measuring the partial pressure of carbon dioxide dissolved in sea water, however, both measuring methods encounter several problems related to the step of forming the gas-liquid equilibrium state and the step of measuring the partial pressure.

(1) With regard to the step of forming the gas-liquid equilibrium state, firstly, the measuring device lacks compactness and it is not practical since measurement cost is high.

More specifically, since both methods require a certain amount of sea water in order to form the droplets or the bubbles to create the gas-liquid equilibrium state, it is impossible, or extremely difficult to directly measure the partial pressure of carbon dioxide in sea water whose level rapidly changes due to waves etc., not only in the shower type but also in the bubble type.

Therefore, if the partial pressure of carbon dioxide is constantly measured at the sea in order to monitor variation in the partial pressure, a container with a certain volume for sampling the sea water, a pipe communicating the container with a given site (the sea level), and a bulky pump for pumping the sea water through the pipe are required.

This inevitably makes the measuring device bulkly and complicated, so that several problems, such as need to adjust the liquid level in the container, blockage of the flow passage, and clogging of the showerhead may arise, and, as a result, the reproducibility of the measurement can be badly affected.

In addition, as discussed further below, from the standpoint of reliability of the measurement, it is necessary to replace the sea water in the container by continuously driving the bulky pump not only before and after the measurement, but also during the measurement. This leads to high electric power consumption that increases the cost of the measurement. Therefore, on the whole, the above method lacks in practicality.

When a liquid having a predetermined partial pressure of some material and a gas having another predetermined partial pressure of the material are passed into an equilibrium cell, the following equation is generally satisfied from the viewpoint of the material balance between before and after the flow of the gas and the liquid.

$$Fg \cdot Cair,in + Fl \cdot Cliq,in = Fg \cdot Cair,out + Fl \cdot Cliq,out$$

Where $Fg$ and $Fl$ are the flow rate of the gas and the liquid, respectively, and $Cair,in$ and $Cair,out$ are the concentrations of the carrier gas flowing in and that flowing out, and $Cliq,in$ and $Cliq,out$ are the concentrations of the liquid flowing in and flowing out.

Presuming that an ideal gas-liquid equilibrium state is formed, the equation can be rewritten as follows.

$$Cair,out = Cliq,out$$

Therefore, the partial pressure of the material after the equilibrium state is determined by the initial partial pressure of the material on the side of the carrier gas and that on the side of the liquid, and the flow rate of the carrier gas and the liquid as well.

Under such a condition, since a finite volume of sea water sample contains a finite amount of carbon dioxide, in the case where the gas-liquid equilibrium state is formed between a finite volume of the carrier gas and a finite volume of sea water, the influence on the equilibrium state of the initial partial pressure of carbon dioxide on the side of carrier gas can be avoided only by either circulating the sea water or restricting the volume and the flow rate of the carrier gas. However, the volume and the flow rate of the carrier gas are determined from the start to enable measurement of the partial pressure by the NDIR device or to form a gas-liquid equilibrium state. The degree to which they can be restricted is therefore limited.

(2) Secondly, the reliability of the measurement value is also a problem. More specifically, the degree of the equilibrium state is bad and there is a discrepancy between the actual value and the measurement value.

Firstly, with regard to the degree of the equilibrium state, if $Fg$ is 200ml/min, $Cair,in$ is 340ppm and $Cliq,in$ is 600 ppm, the flow rate Fl needed to form a semi-complete gas-liquid equilibrium state in which the pressure difference between Cair, out and Cliq,out is within 0.001 ppm is 4m$^3$/min as calculated by the above equation. A pump with such a large capacity is bulky and not practical. On the other hand, if an ordinary pump is used, the pressure difference between Cair, out and Cair, in expands making it extremely difficult to obtain satisfactory results.

The conventional shower system and the bubble system thus achieve only a degree of the equilibrium state and are therefore inferior in measurement reliability.

In order to solve this problem, the present inventor earlier invented a multiple bubble type measuring device.

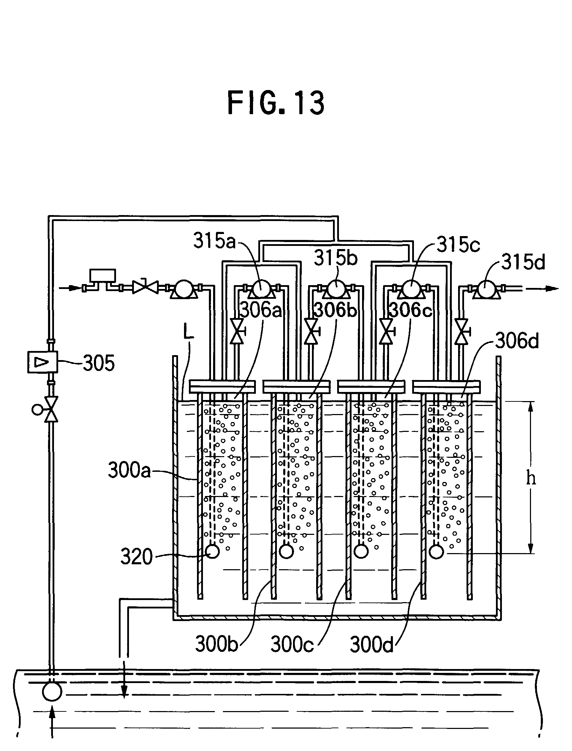

Figure 13 shows a general view of the device for measuring partial pressure of carbon dioxide according to the multiple bubble system. The difference between this and the bubble system shown in Figure 11 is that, in the former, the equilibrium cells are connected in series in multiple stages (four stages, in the illustrated case) and sea water is constantly passed to each of the equilibrium cells.

According to the multiple bubble system, the carrier gas firstly is bubbled in a first equilibrium cell 100a and then delivered to a second equilibrium cell 100b through a carrier gas space 106a formed above a free liquid surface L of the first equilibrium cell 100a. Then, the carrier gas is bubbled again at the second equilibrium cell 100b and this bubbling action is repeated up to a fourth equilibrium cell 100d. This enables a high degree of the equilibrium state to be secured.

Although the multiple bubble system achieves a higher degree of equilibrium state than a single bubble type as shown in Figure 11, the response of the measurement is slow. This system is therefore not practical for continuous measurement of the constantly changing partial pressure of carbon dioxide in sea water.

The time period needed to attain the gas-liquid equilibrium state is critical for the response of the measurement. In this respect, the dominant factors for the response of the measurement are firstly, the dead space around the carrier gas of the measuring device, secondly, the velocity per unit area at which the material is replaced, and thirdly, the area in which the gas and the liquid contact with each other.

This will now be explained by utilizing the equation based on the gas-liquid equilibrium theory. The material movement per unit time from the surface of the bubble balances with the change of concentration per unit time inside the bubble multiplied by the volume thereof. This balance can be mathematically expressed by the following equation.

$$\text{Vair} \cdot \text{dCair/dt} = K \cdot a \cdot (h \cdot \text{Cliq} - \text{Cair})$$

Where Vair is the volume of the gaseous phase, Cair is the partial pressure inside the gaseous phase, t is the time period during which the gas and the liquid contact each other, a is the area of gas and liquid contact, Cliq is concentration of the material inside the liquid phase, h is Henry's law solubility constant, K is an overall mass transfer coefficient.

The following equation is obtained by integrating the above equation.

$$t = -(\text{Vair}/a)(1/K)\ln((\text{Cair} - h \cdot \text{Cliq})/(\text{Ccar} - h \cdot \text{Cliq}))$$

where Ccar is the initial concentration of the carrier gas.

Therefore, in order to make the time t shorter, the volume Vair has to be reduced, or the gas-liquid contacting area has to be increased, or the general coefficient K has to be increased. Under this condition, K is the summation of the coefficient on the side of gaseous phase and that on the side of liquid phase, and the former is negligible since the latter is dominant.

Based on the above, in the prior art shower and bubble systems under discussions, since the size of the dead space and the renewal of the laminar film on the side of the liquid phase in the bubble system are inferior to those in the shower system, the response of the measurement by the bubble system is much faster than that by the shower system.

However, even in the bubble system, as described below, the principle of the measurement limits the improvement that can be achieved in the response rate of the measurement. Namely, when the bubble system is utilized for measurement, in view of the fact that the minimum height of the container is determined based on the formation of the multiple bubbles, which are required for the formation of the gas-liquid equilibrium state and to secure gas-liquid contacting time, a container with a certain volume is needed in the first place. Therefore, in order to speed up the response of the measurement by facilitating the renewal of the surface, which is one of the dominant factors affecting the response, there is no alternative but to enhance the relative speed between the bubbles and the sea water. The relative speed can be increased either by increasing the velocity on the side of the bubbles or the velocity of the sea water flow. However, in the latter case, it is extremely difficult to substantially improve the relative speed. Because the bubbles are so light that they can completely ride on the generated water flow. Accordingly, the only practical way is to increase the upward speed of the bubbles, and thus it is necessary to increase the volume of the gas in order to enhance the floating force exerted on the bubbles inside the sea water.

On the other hand, if the volume of the bubbles is increased, although the coefficient of the material movement can be slightly increased, Vair/ a, another dominant factor affecting the response of the measurement, is automatically increased, meaning that there is a limit to the improvement in response rate possible.

In other words, in the bubble system, since the dominant factors are not independent of one another due to the principle of the measurement, the response can be improved only to a limited degree.

In particular, if the multiple system is adopted, the dead space becomes bigger than that of the single system due to structural factors, so the response becomes worse.

The problem of discrepancy between the actual value and the measurement value is closely related to the bulky pump and the routing of the pipe. Since the equilibrium state depends on the temperature of the sample water, a 1 °C change in water temperature may produce more than a 10 ppm change of the partial pressure of carbon dioxide. If the sample water is pumped by a bulky pump, it is likely to be heated by a pump. It is also apt to be heated or cooled in accordance with the length of the pipe. Development of a discrepancy between the temperature of the sea water in the container and that at the site where the sample water was taken can therefore not be avoided.

In order to compensate for this discrepancy ,it is necessary to provide a correction device which measures the temperature of the water which has reached the gas-liquid equilibrium state and the temperature at the site with high accuracy. This further increases the bulk and complexity of the devices.

(3) Next, with regard to the step of measuring the partial pressure, as shown in Figures 11 and 12, in both systems, dead space is increased due to the provision of the box cell, etc. for measuring the sample gas which has already reached the gas-liquid equilibrium state by pumping the sample gas into the NDIR 130 via the pump 115. The measurement response is badly affected by this increased dead space.

In particular, according to the NDIR method, in order to secure the reliability of the measurement value, not only the sea water but also the carrier gas must be circulated in order to maintain the pressure in the box cell at atmospheric level so as to establish the same condition as that at the site where the sea water is sampled. The liquid level or the pressure therefore has to be controlled in order to keep the pressure of the carrier gas in the container constant. In this respect, it is far more difficult to effect direct measurement at the sea where the sea level varies intensely.

As pointed out above, The conventional device and the method for measuring the partial pressure have two problems. One is inpracticality due to the complexity and bulkiness of the device and the other is the difficulty of ensuring reliability of the result of the measurement. Those skilled in the art see these as interacting problems whose solutions are incompatible.

Especially, since the measurement of the partial pressure of carbon dioxide in sea water requires continuous measurement at the ppm level in view of the object of the measurement, it is strongly desired that these two problems be solved.

Therefore, the object of the present invention is to provide a method and a system for measuring the partial pressure of a material dissolved in a liquid, which enhances the practicality of the measurement while at the same time ensuring the reliability thereof.

Another object of the present invention is to provide a method and a system for measuring the partial pressure of carbon dioxide dissolved in a sea water which, unlike the conventional bubble and shower systems, enhances practicality while at the same time increase a response of the measurement by direct measurement inside the sea water when the partial pressure of carbon dioxide dissolved in sea water is continuously measured.

SUMMARY OF THE INVENTION

These and other objects are achieved according to the present invention in one aspect thereof by providing a method for measuring partial pressure of a material in a liquid including a step of forming a gas-liquid equilibrium state between the liquid in which the material is dissolved and a carrier gas, and a step of measuring the partial pressure of the material dissolved in the liquid by utilizing vaporized material in the carrier gas which has reached the gas-liquid equilibrium state, characterized in that the forming step includes a step of forming the gas-liquid equilibrium state between the liquid and the carrier gas present within a certain volume through a free liquid surface existing at each hole of a hydrophobic porous membrane including holes, the diameter and total area of the holes being such that the liquid cannot permeate through the hydrophobic porous membrane due to its surface tension but the material can permeate therethrough.

According to the above constitution, when the hydrophobic porous membrane which is perforated with holes each of a prescribed diameter and whose holes have a prescribed total area is introduced into a liquid in which a material is dissolved, the liquid is blocked at each of the holes due to its surface tension but the material can permeate through each of the holes. A gas -liquid equilibrium state of the material is therefore formed between the liquid and the predetermined volume of the carrier gas through the free liquid surface. As a result, the partial pressure of the material in the liquid can be measured by utilizing the vaporized material in the carrier gas which has reached the gas -liquid equilibrium state.

In this case, unlike in the case of the conventional shower system or the bubble system, since the measurement can be directly conducted in the liquid without the liquid leaking into the carrier gas chamber, no container for sampling the liquid and no pump for constantly pumping the liquid into the container during measurement are needed. The measurement device can therefore be made compact and simple and the cost of measurement can be reduced. The practicality of the method is therefore high.

On the other hand, as described below, on the whole, the reliability of the measurement can be enhanced by adjusting the response of the measurement and the reduction of noise, a cause for discrepancy between the actual value and the measurement value, thanks to the elimination of the pump.

Table 1 shows a comparison between the characteristics of the dominant factors for affecting the response in the conventional shower and bubble systems and those in the porous membrane systems according to the present invention.

## Table 1

## Comparison of dominant factors

## related to the response in different systems

| Dominant Factors Type | | Dead Space | Coefficient of material movement on side of liquid | Area per unit volume of carrier gas in which gas and liquid contact |
|---|---|---|---|---|
| Prior Art | Bubble | Fair | Fair | Good |
| | Shower | Poor | Poor | Fair |
| Present Invention | Porous Membrane | Good | Good (controllable) | Good (controllable) |

The greatest decrease in the volume of the dead space, the first factor is achieved by porous membrane system, followed by the bubble system and the shower system in that order. This is because, in the bubble and shower systems, the container (the container 100 in Figures 11 and 12) for forming the gas-liquid equilibrium state has to be large, the gas-liquid equilibrium state is formed during the time period while the bubbles rise or the droplets fall, whereas, in the porous membrane system, the only unavoidable dead space associated with the formation of the gas-liquid equilibrium state is the carrier gas chamber.

The porous membrane system can thus enhance response by reducing dead space.

The greatest increase in the coefficient of the material movement, the second factor, is achieved by the porous membrane system followed by the bubble type and the shower type in that order. In particular, in a case of the porous membrane system, the coefficient of the material movement can be adjusted by breaking or renewing the laminar film on the side of the liquid.

As to the gas-liquid contact area per unit volume of the carrier gas between the gas and the liquid, the third factor, although the shower system is inferior to the other two types as pointed out above, there is no substantial difference between the porous membrane and bubble systems. However, in the porous membrane system, the third factor can, like the second factor, be adjusted. This can be achieved by adjusting the total area of the holes. The surface area of the bubbles cannot be adjusted in the bubble system.

In particular, in the porous membrane system according to the present invention, the coefficient of the material movement (the second factor) can be adjusted independently by the gas-liquid contact area per unit volume of the carrier gas (the third factor).

Therefore, according to the present invention, the partial pressure of carbon dioxide in sea water, which changes momentarily, can be monitored at ppm level.

In addition, when the material dissolved in the liquid is carbon dioxide dissolved in sea water, the gas-liquid equilibrium state can be formed by directly introducing the hydrophobic porous membrane into the sea water. By such direct measurement of the partial pressure in the sea water, the influence on the partial pressure of carbon dioxide in the sea water of absorption and discharge of carbon dioxide by the carrier gas in the carrier gas chamber can be eliminated because the membrane contacts a great amount of sea water.

These and other objects are achieved according to the present invention in another aspect thereof by providing a system for measuring partial pressure of carbon dioxide dissolved in a liquid including means for forming a gas-liquid equilibrium state of carbon dioxide between the liquid in which carbon dioxide is dissolved and the carrier gas, and means for measuring the partial pressure of carbon dioxide dissolved in the carrier gas which has reached the gas-liquid equilibrium state, characterized in that

said forming means includes a hydrophobic porous membrane having holes, the diameter and the total area of the holes being such that the liquid cannot permeate through the hydrophobic porous membrane due to its surface tension but carbon dioxide can permeate therethrough, said membrane includes an inner membrane surface contacting the carrier gas and an outer membrane surface contacting the liquid, said forming means further includes a carrier gas chamber open into the inner membrane surface including an inlet opening through which the carrier gas flows in and an outlet opening through which the carrier gas flows out, whereby a gas-liquid equilibrium state is formed between the carrier gas inside the carrier gas chamber and the liquid through the free liquid surface existing at each hole.

According to the above constitution, when the hydrophobic porous membrane having holes of prescribed diameter and prescribed total area is introduced into the liquid, the liquid is blocked at the holes due to its surface tension but the carbon dioxide can pass through the holes. A gas-liquid equilibrium state can therefore be formed through the free liquid surface, so that the partial pressure of carbon dioxide dissolved in the liquid can be measured by utilizing the carbon dioxide gas in the carrier gas which has reached the gas-liquid equilibrium state.

More specifically, since the hydrophobic porous membrane has hydrophobicity, the liquid cannot pass through the holes due to its surface tension. As a result, a free liquid surface is formed at each hole, On the other hand, carbon dioxide reaches the inner surface of the membrane through the holes via the free liquid surface. After carbon dioxide reaches the carrier gas flowing into the carrier gas chamber from the inlet, the gas-liquid equilibrium state can be formed between the carrier gas in the carrier gas chamber so that the partial pressure of carbon dioxide inside the carrier gas can be measured by means for measuring the partial pressure of carbon dioxide utilizing the carrier gas flowing out of the outlet.

In this case, unlike in the conventional shower and bubble systems, since measurement can be directly performed in the sea water without the liquid leaking into the carrier gas chamber, no container for sampling the liquid and no pump for constantly pumping the liquid to this container during measurement are needed. This enhances practicality by enabling the measurement system to be made compact and simple, and also reduces the cost of measurement.

On the other hand, as in the first aspect of the invention, the adjustment of the measurement response and the elimination of the pump, a source of noise that is a cause for discrepancy between the actual value and the measurement value, enhances the reliability of the measurement on the whole.

The system preferably further includes means for pumping the liquid toward said outer membrane surface to renew the laminar film on the side of the liquid phase adjacent to said outer membrane surface of said hydrophobic porous membrane.

The hydrophobic porous membrane is preferably coated with a hydrophobic material.

The forming means preferably further includes means disposed on the inner membrane surface inside said carrier gas chamber for supporting the said hydrophobic porous membrane in the direction of the outer membrane surface.

It is also preferable that said supporting means includes a plurality of beads of predetermined diameter and the plurality of beads are disposed to contact each other over the entire inner membrane surface, whereby a flow passage for the carrier gas is formed from said inlet opening to said outlet opening of the carrier gas chamber through gaps between the adjacent beads.

Preferably the outlet opening is disposed remote from the inlet opening and a labyrinth is formed by said carrier gas passage.

The system also preferably further includes multiple partitions around said outlet opening to guide the carrier gas from said inlet opening to said outlet opening, each partition including an opening located so that the openings of adjacent partitions are diametrically opposite from each other, whereby the labyrinth is formed by said carrier gas flow passage defined between the adjacent partitions.

The system also preferably further includes a spiral partition extending from said inlet opening to said outlet opening to guide the carrier gas, whereby said labyrinth is formed by a serpentine carrier gas flow passage defined along said spiral partition.

The measuring means preferably consists of a non-dispsersive infrared gas analyzer including a box cell through which sample gas having reached gas-liquid equilibrium state flows, a light source for introducing light into the box cell, and a photodetector for detecting the intensity of introduced light which is absorbed by carbon dioxide inside the sample gas.

The measuring means preferably includes a circulation passage between the box cell and the carrier gas chamber and a circulating pump for circulating the carrier gas via the circulation passage.

A fan for mixing the carrier gas inside the carrier gas chamber is preferably provided inside said carrier gas chamber.

The hydrophobic porous membrane is preferably disposed to cover an opening provided on a side of said box cell, whereby a gas-liquid equilibrium state is formed inside the box cell.

The foregoing and other objects are achieved according to the present invention in still another aspect thereof by providing a system for measuring partial pressure of carbon dioxide dissolved in the liquid including means for forming a gas-liquid equilibrium state of carbon dioxide between the liquid in which carbon dioxide is dissolved and a carrier gas, and means for measuring the partial pressure of carbon dioxide dissolved in the carrier gas which has reached the gas-liquid equilibrium state, characterized in that

said forming means includes a hydrophobic porous membrane having holes of prescribed diameter and total area, said membrane includes an inner membrane surface contacting the carrier gas and an outer membrane surface contacting the liquid, a gas-liquid equilibrium state being formed between the liquid and air remaining in the individual holes via the free liquid surface existing at each hole, and said measuring means includes a solid electrolyte sensor disposed on the side of the inner membrane surface for measuring partial pressure of carbon dioxide by detecting an electromotive force developed by carbon dioxide in the air.

According to the above constitution, when the hydrophobic porous membrane with holes of prescribed diameter and total area is introduced into a liquid in which carbon dioxide is dissolved, the liquid is blocked at the holes due to its surface tension but the carbon dioxide can pass through the holes. A gas-liquid equilibrium state can therefore be formed between the extremely small amount of air remaining in the individual holes and the liquid via the free liquid surface, so that the partial pressure of carbon dioxide dissolved in the liquid can be measured by utilizing the measuring means to measure the carbon dioxide gas in the air which reached the gas-liquid equilibrium state.

More specifically, since the porous membrane has hydrophobicity, the liquid cannot pass through the holes due to its surface tension. A free liquid surface can therefore be formed at each hole. On the other hand, the carbon dioxide reaches the inner surface of the membrane through the free liquid surface. The gas-liquid equilibrium state can therefore be formed between the air remaining in each of the holes and the liquid. The partial pressure of carbon dioxide dissolved in the liquid can therefore be measured by utilizing the air which has reached the gas-liquid equilibrium state and detecting the electromotive force generated by carbon dioxide gas in the air with the solid electrolyte sensor.

In this case, unlike in the conventional shower and bubble systems, the partial pressure can be directly measured without the liquid leaking into the carrier gas chamber and, in addition, no container for sampling the liquid or pump for constantly pumping the liquid into this container during the measurement needed to be provided. As a compact sensor-type system for measuring partial pressure of carbon dioxide can therefore be realized, a measuring system can be obtained that is small in size, simple, low in measurement cost and, accordingly, excellent in overall practicality.

On the other hand, as in the first and second aspects of the invention, the reliability of the measurement can be improved on the whole by eliminating the pump as a source of noise that is a cause for the discrepancy between the actual value and the measurement value.

A thermal shield plate preferably is disposed between the porous membrane and the solid electrolyte sensor.

The above and other objects, features, and advantages of the present invention will become apparent from the following description read in conjunction with the accompanying drawings, in which like reference numerals designate the same elements.

BRIEF DESCRIPTION OF THE DRAWINGS

Figure 1 is a schematic diagram showing a system for measuring partial pressure of carbon dioxide according to a first embodiment of the present invention.

Figure 2 is a schematic sectional view showing an equilibrator of the system for measuring partial pressure of carbon dioxide in Figure 1.

Figure 3 is a schematic sectional view taken along a line 2-2 of Figure 1, Figure 3(a) showing the equililbrator of type 3 and Figure 3(b) showing those of types 1 and 2, (beads inside the equilibrators are partially omitted for clarity of the drawing).

Figure 4 is a graph showing time-course change in partial pressure of carbon dioxide measured by a system combining an equilibrator of type 3 and a hydrophobic porous membrane of type A.

Figure 5 is a graph similar to Figure 4 showing time-course change in partial pressure of carbon dioxide measured by a system combining an equilibrator of type 3 and a hydrophobic porous membrane of type B.

Figure 6 is a graph similar to Figure 4 showing time-course change in partial pressure of carbon dioxide measured by a system combining an equilibrator of type 3 and a hydrophobic porous membrane of type C.

Figure 7 is a graph similar to Figure 4 showing time-course change in partial pressure of carbon dioxide measured by a system combining an equilibrator of type 2 and a hydrophobic porous membrane of type C.

Figure 8 is a graph similar to Figure 4 showing time-course change in partial pressure of carbon dioxide measured by a system combining an equilibrator of type 1 and a hydrophobic porous membrane of type C.

Figure 9 is a schematic sectional view showing an equilibrator according to a second embodiment of the present invention.

Figure 10 is a schematic view showing a system for measuring partial pressure according to a third embodiment of the present invention.

Figure 11 is a schematic view showing a conventional bubble system for measuring partial pressure of carbon dioxide.

Figure 12 is a schematic view showing a conventional shower system for measuring partial pressure of carbon dioxide.

Figure 13 is a schematic view showing a conventional multiple bubble system for measuring partial pressure of carbon dioxide.

DESCRIPTION OF THE PREFERRED EMBODIMENTS

Preferred embodiments of the system and the method for measuring the partial pressure of a material, carbon dioxide in the embodiments, according to the present invention will now be described by way of example with reference to the drawings. A first embodiment according to the present invention will now be described in detail.

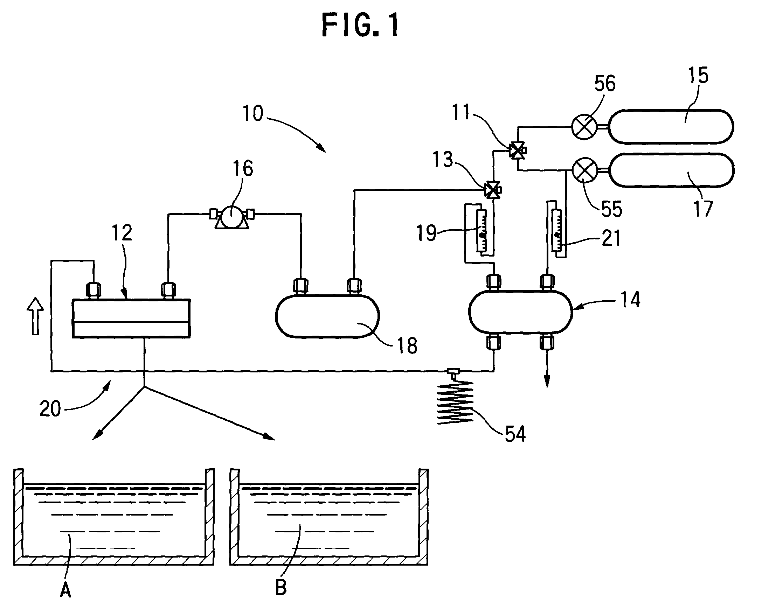

As shown in Figure 1, measuring the partial pressure of carbon dioxide incorporating different combination of an equilibrator and a hydrophobic porous membrane were tested for response and measurement reproducibility alternately using distilled water A and distilled water B respectively containing carbon dioxide with high and low partial pressure.

The system for measuring partial pressure of carbon dioxide, designated by reference numeral 10, comprises an equilibrator 12 constituting means for forming a gas-liquid equilibrium state, an NDIR 14 constituting means for measuring the partial pressure of carbon dioxide, an air pump 16 and an electronic dehumidifier 18 interposed between the equilibrator 12 and the NDIR 14, and piping 20 by means of which a carrier gas is circulated through the equilibrator 12, the NDIR 14, the air pump 16 and the electronic dehumidifier 18. The carrier gas is air.

Firstly, the equilibirator 12 constituting the means for forming a gas-liquid equilibrium state will be described.

The table 2 sets out the specificatious three equilibrators 12.

### Table 2

### Specification of equilibrators

| Type / Items | Equilibrator 1 | Equilibrator 2 | Equilibrator 3 |
|---|---|---|---|
| Bead diameter | 2mm | 3mm | 3mm |
| Groove depth | 2.5mm | 3.5mm | 3.5mm |
| Membrane area | $226cm^2$ | $226cm^2$ | $226cm^2$ |
| Dead space | $28cm^3$ | $40cm^3$ | $40cm^3$ |
| Labyrinth | No | No | Yes |

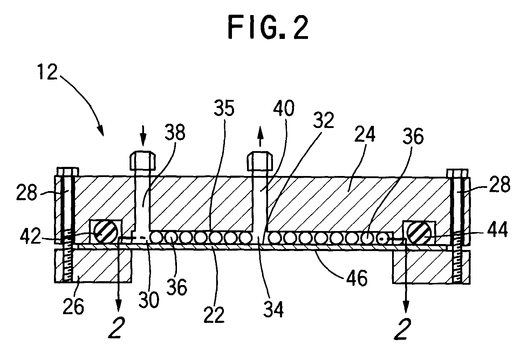

As shown in Table 2, three types ( type 1 to type 3 ) of the equilibrator 12 classified in accordance with the diameter of beads 36 and the presence of a labyrinth described later were adopted. Figure 2 shows the type 3 equilibrator which includes a labyrinth. As shown in Figure 2, the profile of the type 3 equilibrator 12 is a substantially cylindrical and a hydrophobic porous membrane 22 described below is sandwiched between a body 24 and a retainer ring 26 and fixed by a fixing 28. The body 24 is provided on its lower surface with a circular recess 30 which forms a carder gas chamber 34 facing an inner membrane surface 32 of the hydrophobic porous membrane 22. The recess 30 includes a substantially planar bottom surface 35 and the depth to this bottom surface 35 is slightly larger than the diameter of beads 36 described below disposed in the recess 30, as shown in Table 2. The recess 30 is provided with, an inlet 38 through which the carrier gas flows in and an outlet 40 through which the carder gas flows out. The outlet 40 is disposed substantially at the center of the recess 30. The carrier gas flowing into the carrier gas chamber 34 from the inlet 38 forms the gas-liquid equilibrium state via the free liquid surface created due to its surface tension at each hole in the hydrophobic porous membrane 22 described below, and then flows out from the outlet 40 to the NDIR 14. An annular groove 42 for mounting an O-ring 44 is provided around the circular recess 30. The O-ring 44 is disposed inside the annular groove 42 so as to seal the interface between the hydrophobic porous membrane 22 and the body 24 in order to prevent the liquid from leaking into the carrier gas chamber 34. The retainer ring 26 is fixed to the peripheral portion of the hydrophobic porous membrane 22 so as not to cover the holes of the hydrophobic porous membrane 22.

A fan (not shown) is provided inside the carrier gas chamber 34 for mixing the carrier gas in the carrier gas chamber 34 in order to efficiently form the gas-liquid equilibrium state.

In addition, the equilibrator 12 includes a number of beads 36 of prescribed diameter disposed on the inner membrane surface 32 inside the carrier gas chamber 34. These beads 36 are so disposed as to contact each other over the entire inner surface 32, and have a spherical shape. Therefore, when the device 10 is introduced into the liquid, they can support the membrane against the liquid pressure acting on the membrane outer surface 46, maintain an adequate effective area of the membrane, and ensure passage of the carrier gas. Namely, the point contact of the beads 36 with the inner membrane surface 32 minimizes closure of the holes of the hydrophobic porous membrane 22, while the point contact between adjacent beads establishes gaps among the beads that form a passage for the carrier gas. In addition, these beads 36 have a function of improving the response of the measurement by reducing the dead space inside the carrier gas chamber 34.

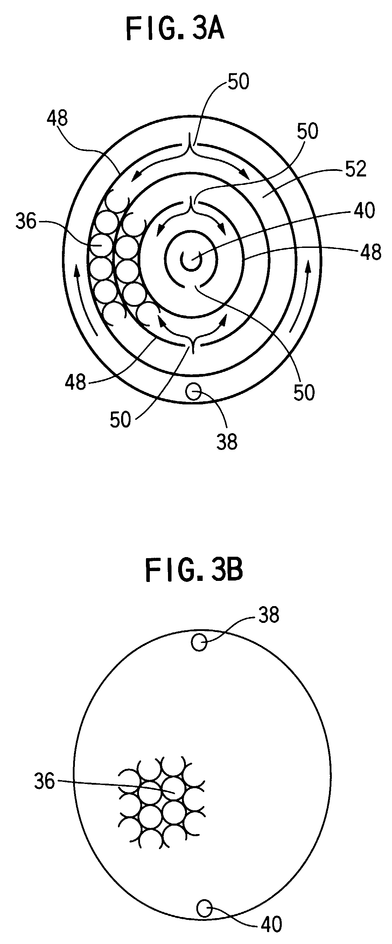

As shown in Figure 3(A) (which depicts only part of the total number of beads in the interest of clarity), in the case of the type 3 equilibrator including the labyrinth, four annular partitions 48 are disposed around the outlet 40 and fixed to the bottom surface 35 in order to guide the carrier gas from the inlet 38 to the outlet 40. Each of partitions 48 includes an opening 50 and the adjacent partitions 48 form annular passages 52 for the carrier gas in the manner of a labyrinth. The carrier gas flowing into the carrier gas chamber 34 from the inlet 38 flows toward the outlet 40 through the gaps between the beads adjacent to each other along the annular passages 52, as shown by arrows. This prevents short-circuiting and ensures that the gas-liquid equilibrium state can be efficiently formed by securing an adequate time period during which the carrier gas contacts the free liquid surfaces present at the holes.

On the other hand, Figure 3(B) (which depicts only part of the total number of beads in the interest of clarity) shows an equilibrater 12 of Type 1 or 2, without a labyrinth. This type of equlibrator 12 includes an outlet 40 which is disposed remote from the inlet 38 in the carrier gas chamber 34. A labyrinth is formed from the inlet 38 to the outlet 40 through gaps (not shown) between adjacent beads 36 by disposing the outlet 40 diametrically opposite from the inlet 38. Alternatively, in order to guide the carrier gas from the inlet 38 to the outlet 40, a spiral partition extending from the inlet 38 to the outlet 40 may be provided for forming a labyrinth by means of a serpentine carrier gas flow passage defined along the spiral partition.

Table 3 sets out the specifications of the hydrophobic porous membrane 22.

## Table 3

## Specifications of membranes

| Type<br>Items | A | B | C |
| --- | --- | --- | --- |
| | PALL<br>$1.2\mu$ | PALL<br>$0.2\mu$ | MILLIPORE<br>$1.0\mu$ |
| Product Designation | Hydrolon P/N HNNH8115, Lot.094641 | Hydrolon P/N HNRHH-SS, Lot.244540 | Dulapel Membrane ultra-hydrophobic PVDF PFIJ003E4 |
| Hole Diameter | $1.2\mu$ | $0.2\mu$ | $1.0\mu$ |
| Void volume | No data | No data | 70% |
| Thickness | $130\mu$ | $130\mu$ | $125\mu$ |
| Material | Nylon 66 | Nylon 66 | Hydrophobic polyvinyl fluoride |

The hydrophobic porous membrane 22 can be a commercially available thin circular membrane. Three types (type A to type C) classified in accordance with the kind of the membrane, the thickness thereof and the diameter of the holes are shown in Table 3 by way of example. As shown in Figure 2, the hydrophobic porous membrane 22 includes the inner membrane surface 32 contacting the carrier gas, an outer membrane surface 46 contacting the liquid and a number of holes ( not shown ) each of which permeates from the inner membrane surface 32 to the outer membrane surface 46. The diameter of each hole and the total area of the holes are determined in view of the material to be measured (carbon dioxide, in this embodiment) and the time it takes to form the gas-liquid equilibrium state. The material of the membrane is resistant to corrosion. It also has a degree of hydrophobicity such that distilled water is prevented from permeating through the holes by its surface tension but carbon dioxide is able to permeate though the holes.

In addition, since shorter the length of the holes is better for faster response, the porous membrane is a thin film on the order of 100 μm. If necessary to obtain the required resistance to corrosion and hydrophobicity, the surface of the membrane may be coated with a coating providing resistance to corrosion and hydrophobicity.

Next, means for measuring the partial pressure of carbon dioxide, the NDIR14 will be explained. The NDIR 14 is of a conventional type comprising a box cell through which the sample gas having reached the gas-liquid equilibrium state passes, a light source for directing light into this box cell and a photodetector for detecting the intensity of the light absorbed by carbon dioxide in the sample gas. Therefore, further explanation about the NDIR 14 is omitted. The inside of the box cell is separated and thus divided into a reference cell and a sample cell (both not shown). Table 4 shows the NDIR test conditions.

Table 4

| Measuring conditions of NDIR system | |
| --- | --- |
| Type of NDIR | LI-COR6251 |
| Circulating flow Of carrier gas | 300ml/min |
| Concentration of zero gas For calibrating NDIR | Air+$CO_2$ 328.88 ppm |
| Concentration of the span gas For calibrating NDIR | Air+$CO_2$ 498.25 ppm |
| Time period of Equilibrator insertion | 15 minutes |
| Metering period | 1 second |
| Data sampling period | 5 seconds (the average data) |

With a view to preventing the measurement from being affected by the fact that the carrier gas flowing into the box cell pressurizes the inside of the box cell, a three-way tube 54 through which the box cell is communicated with the outside is provided near the discharging side of the NDIR in order to balance the pressure in the carrier gas with the ambient pressure. As shown in Figure 1, the NDIR14 is calibrated by a zero gas 11 and a span gas 15 (Table 4) by switching solenoid valves 11, 13. When the ambient pressure varies during the measurement test, the zero gas 17 is constantly passed into the NDIR 14.

Water vapor present in the carrier gas tends to absorb infrared rays whose wavelengths overlapping those absorbed by carbon dioxide. The electronic dehumidifier 18, which is of conventional type, is therefore disposed upstream relative to the NDIR 14 in order to dehumidify the carrier gas before the carrier gas flows into the NDIR.

The piping 20 forms a circulation passage for circulating the sample gas between the box cell and the carrier gas chamber 34 and is configured in such a way that the sample gas is circulated through this circulation passage by the air pump 16. Piping of small diameter is preferable, because the smaller the diameter of the pipe, the smaller the dead space becomes. The material of piping is preferably Cu, SUS or plastic, which has small tendency to absorb the gas.

As described above, the system 10 for measuring the partial pressure according to this embodiment can measure the partial pressure by directly introducing the equilibrator 12 into the liquid to be measured. The system can be therefore be realized in a simpler and more compact configuration than the conventional bubble or shower system.

The operation of the device 10 for measuring partial pressure comprising the above components will be described along with a test procedure. The explanation will be made with respect to a system equipped with a type 2 equilibrator 12 as an example.

(1) Firstly, a stand-by condition for metering is set by calibrating the NDIR 14. This will be explained in more detail below by dividing the process into a calibration process consisting of a zero calibration and a span calibration, and the metering process.

At first, the zero gas 17 is divided into two branches downstream of a pressure relief valve 55. The zero gas is passed into the reference cell inside the NDIR by one of the branches at a constant flow rate of 20 ml/min in order to counteract the variation in atmospheric pressure during the test. The flow rate is detected by a flow meter 21 and the flow is controlled by a needle valve ( not shown ) attached to the flow meter. When the NDIR has been calibrated, the other of the branch is communicated with the sample gas inside the NDIR by switching the flow passage using the solenoid valves 11, 13. The zero calibration is conducted by this procedure and the gas is exhausted through a three-way tube 54 which is open to atmosphere.

Next, the span gas 15 is passed through a pressure relief valve 56 and to the sample cell inside the NDIR by switching the flow passage using the solenoid valves 11, 13. The span calibration is carried out by this procedure and the span gas is again exhausted through the three-way tube 54.

After the above calibration procedure is completed, the span gas 15 and the zero gas 17 are shut off by the solenoid valve 11 and the solenoid valve 13 is simultaneously switched. As a result, a circulation circuit comprising the equilibrator 12, the NDIR 14, the electronic dehumidifier 18 and the pump 16 is formed, whereby the metering stand-by condition for metering is established. The flow rate is detected by the flow meter 19 and the flow is controlled to around 200 to 300 l/min by the needle valve ( not shown ) attached to the flow meter. The total dead space affecting the response of the measurement including that in the NDIR 14, the pipe 20, the equlibritaor 12 and the like, is 80cm$^3$.

(2) For testing, distilled water A and distilled water B with high and low partial pressures are prepared in advance by bubbling. Since the equlibrator 12 disposed in the atmosphere is equilibrated with the atmosphere through the hydrophobic porous membrane 22, the NDIR 14 initially indicates the partial pressure in the atmosphere.

(3) Then, the equilibrator 12 is introduced into the distilled water A having high partial pressure with the outer membrane surface 46 oriented downwardly and substantially parallel with the liquid surface. The liquid is blocked at each hole of the hydrophobic porous membrane 22 due to its surface tension enhanced by the hydrophobicity of the membrane. This causes a gas-liquid equilibrium state to be formed between the liquid in which the carbon dioxide is dissolved (the liquid phase side) and the carrier gas (the gaseous phase side) via the free liquid surface at each of the holes. Namely, carbon dioxide in the liquid permeates through the holes of the membrane 22 to the inner membrane surface 32, and then passes into the carrier gas chamber 34 at the inner membrane surface 32. The carrier gas passed into the carrier gas chamber 34 from the inlet 38 flows out of the outlet 40 via the labyrinth while forming the gas-liquid equilibrium state.

(4) The carrier gas containing carbon dioxide flows via the air pump 16 into the electronic dehumidifier 18 where the vapor in the carrier gas is removed, and reaches the NDIR 14. Utilizing the carrier gas having formed the gas-liquid equilibrium state, that is, the sample gas, the partial pressure of carbon dioxide in the liquid is measured under the conditions of metering period and data sampling period shown in Table 4. After the measurement, the carrier gas is returned to the equilibrator 12 via the piping 20, and the same cycle as described above is repeated.

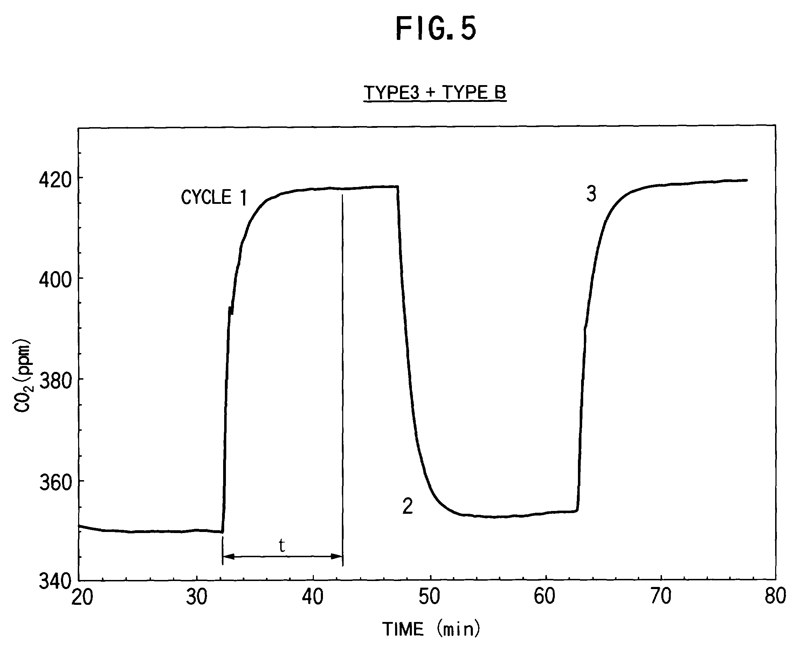

As a result, gas-liquid equilibrium state is formed in the carrier gas chamber 34 while the equilibrator is immersed (15 minutes). And as a result, the NDIR 14 value approaches the partial pressure of the distilled water A and then becomes constant. The value indicated by the NDIR at this time is the final result of the measurement. The value is considered to be constant if its variation is within 0.5 ppm per minute. As shown in Figure 5, time T which it takes to reach this constant value is defined as the response.

(5) Then, the equilibrator 12 is introduced into the distilled water B with low partial pressure or allowed to stand in the atmosphere, whereafter the response and the indicated value are determined in the same way as for the distilled water A.

(6) The response and the reproducibility are evaluated by repeating the above processes (1) to (5).

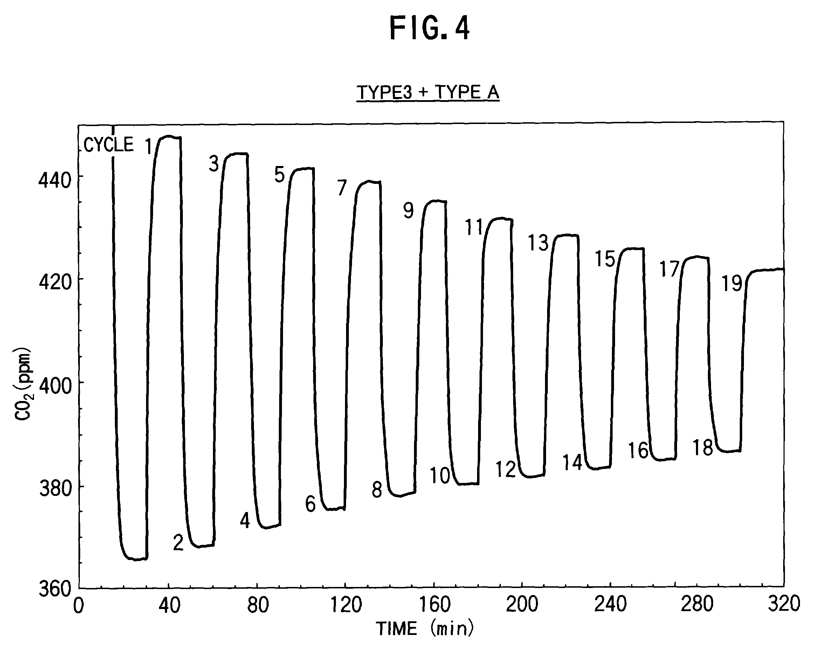

The results of tests conducted by the inventor are shown in Figures 4 to 8.

Each of Figures 4 to 8 is a graph showing time-course change of the partial pressure of the carbon dioxide measured by the device for measuring the partial pressure of carbon dioxide shown in Figure 1. The combinations of the equilibrator with the hydrophobic porous membrane adopted in the tests are type 3+type A, type 3+type B, type 3+type C, type 2+type C and type 1+ type C.

Regarding reproducibility

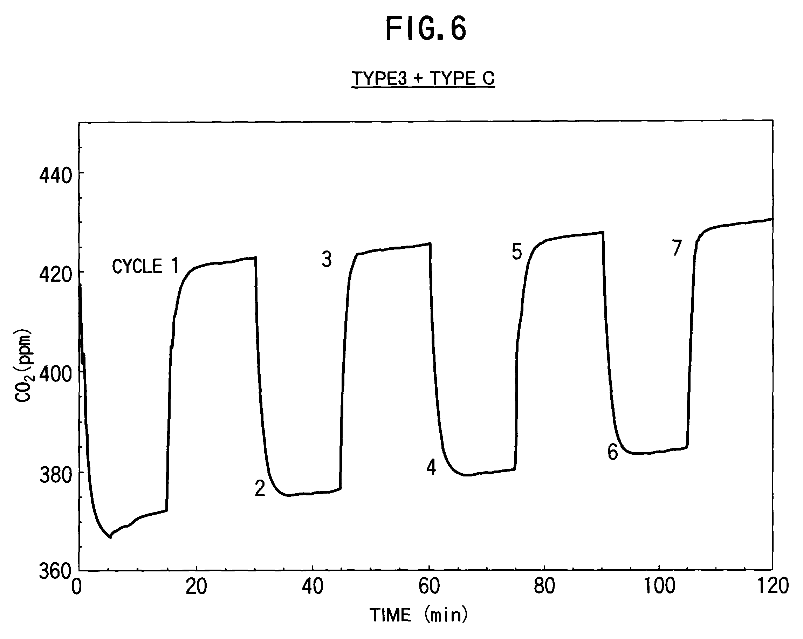

As seen in Figures 4 to 8, the partial pressure of the distilled water A with high partial pressure and that of the distilled water B with the partial pressure varied over time, with the repetition of the cycle. In particular, Figure 4 shows that the partial pressure of the distilled water B increased with passage of time but that, the partial pressure of the distilled water A with high partial pressure decreased with passage of time. On the other hand, Figure 6 shows that the partial pressures of both tended to increase.

As explained in the following, however, this does not indicate a problem in the reproducibility of the measurement. In measurement at the ppm level, even a variation in the partial pressure of carbon dioxide in the air caused by breathing of the operator or by air entering from outside can influence the partial pressure of the distilled waters A and B. Namely, since each of the containers is open to the atmosphere, the distilled water absorbs and discharges carbon dioxide in the atmosphere. As a result, the partial pressures of the distilled waters A and B to be measured vary over time.

The partial pressure of carbon dioxide in the air within the room when the test was carried out was about 400 ppm in the test of Figure 4, whereas it was more than 440 ppm in the test of Figure 6. The results shown were obtained because the partial pressures gradually drew near the partial pressure in the room.

Regarding the influence of the hydrophobic porous membrane on the response rate

Figures 4 to 8 show that the average response as defined in the above manner was about 6.8 minutes, 7.0 minutes, 5.3 minutes, 6.6 minutes and 7.3 minutes, all of which indicate that the practicality and the reproducibility of the measurement by this measuring method. The average of response rate is defined as the average of the response rates of the individual cycles. In the conventional bubble and shower systems, the response rate is more than 10 and 30 minutes, respectively. The quickest response was realized when the diameter of the beads was 3 mm and an equilibrator 12 of type 3 with labyrinth was used.

A comparison of Figure 4 with Figure 5 does not reveal any effect of hole diameter of the hydrophobic porous membrane 22 on the speed of the response. In particular, in Figure 4, although the variation range of the partial pressure decreased over time, the speed of the response rate did not increase. Specifically, no tendency for the response rate to increase was observed. Further, a comparison of Figures 4 and 5 with Figure 6 shows that the response rate was affected more by the kind of the hydrophobic porous membrane 22 than by the diameter of the holes. Therefore, the hole diameter needed only be determined in such a way that the liquid is prevented from permeating through the holes by its surface tension.

Regarding the influence of beads on the response

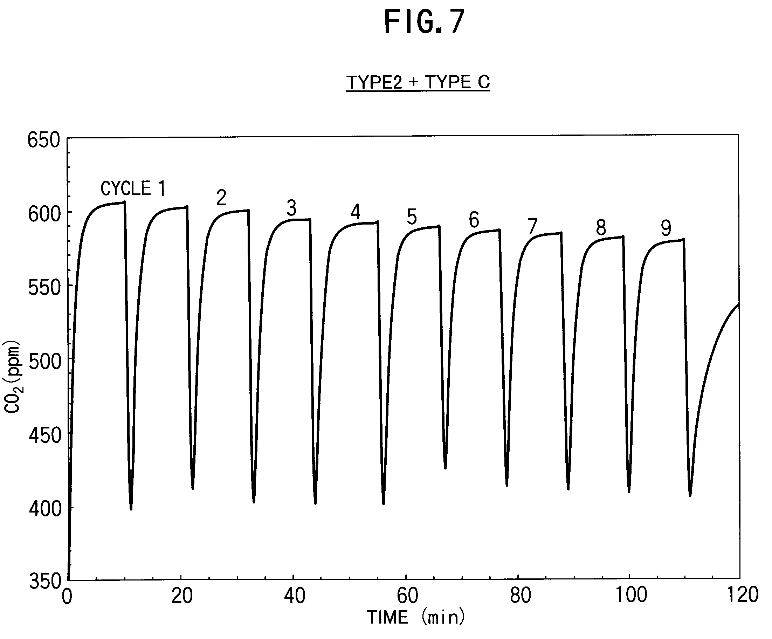

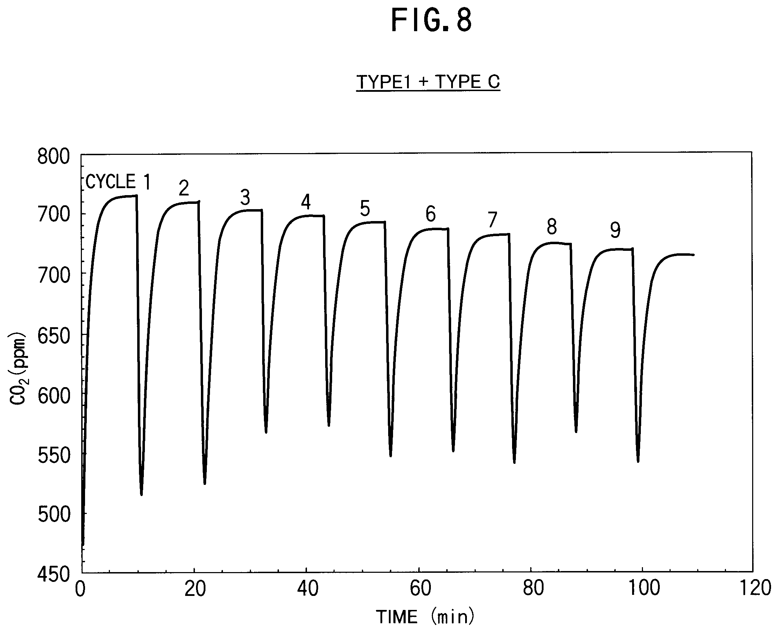

When Figure 7 is compared with Figure 8, it is found that the bigger the diameter of the beads 36, the faster the response became. Although a smaller the diameter of the beads 36 means that less dead space V is formed by the gaps between adjacent beads, on the other hand it means that the total number of beads 36 per unit membrane area increases to increase the total area of contact with the membrane and reduce the total area of the holes contributing to the formation of the gas-liquid equilibrium state.

It was thus found that the ratio of the total area S to the dead space V is an important factor in determining the diameter of the beads.

The tests of Figures 7 and 8 were conducted by alternately and repeatedly introducing the equilibrator 12 into the distilled water A with high partial pressure and letting it stand in the atmosphere.

As can be dearly seen from the above results, although the response rate by the device according to this embodiment varied depending on the kind of the membrane, it always turned out to be less than 10 minutes. Besides, since the reproducibility of the measurement was satisfactory, the test results demonstrate that the device according to this embodiment is highly practical.

A second embodiment of the present invention will now be described. The components of this embodiment similar to those of the first embodiment are indicated by the same reference numbers and will not be described further. Thus, only the features of the second embodiment that differentiate it from the first embodiment will be described hereinafter.

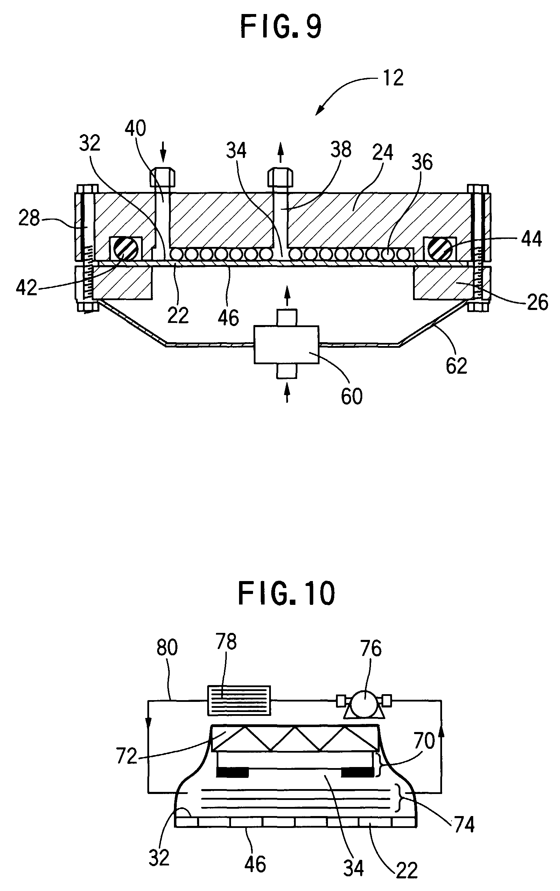

Figure 9 is a general view showing the equilibrator 12 according to the second embodiment. The technical feature of this embodiment lies in the fact that a compact pump 60 is provided under the membrane 22 via a fixing stay 62. When partial pressure is measured, the equilibrator constituting the means for forming the gas-liquid equilibrium state is introduced into water. It has been found that since the diffusion coefficient of the liquid is low when there is no flow in the liquid, a laminar film occurs on the side of the liquid, in which the gradient of the partial pressure is high, at the portion of contact between the liquid and the hydrophobic porous membrane 22, so that the response is considerably slowed.

In order to solve this problem, the compact water pump 60 is provided when the system is used in an environment where there is no water flow, such as in a pond, a swamp, etc. The water pump 60 causes the liquid to flow onto the outer membrane surface 46, thereby increasing the diffusion coefficient of the liquid to prevent response degradation, and can be operated with minimum electric power consumption. Unlike the pumps used in the bubble or shower systems, the water pump 60 is required only to generate near the porous membrane 22 sufficient to renew the laminar film on the side of the liquid. Its electric power consumption is therefore naturally small.

Alternatively, there can be utilized a unitary NDIR that integrates the equilibrator 12 and the NDIR 14 into a single compact unit. In this alternative system, it is preferable that the gas-liquid equilibrium state can be directly formed inside the box cell by disposing the hydrophobic porous membrane 22 so as to cover an opening provided on the side of the box cell. Unlike the separate type, such as in the first and second embodiments, this enables unmanned measurement to be conducted by mounting the measurement device on a buoy floating in the sea, for instance.

A third embodiment according to the present invention will now be described.

Figure 10 is a general view showing the system for measuring partial pressure according to the third embodiment of the present invention. The technical feature of this embodiment lies in the means for measuring the partial pressure of the carbon dioxide inside the carrier gas which has reached the gas-liquid equilibrium state by the means for forming the gas-liquid equilibrium state. Namely, unlike the cases of the first and second embodiments, a solid electrolyte sensor is adopted for such measuring means.

This solid sensor, known as a NASICON, can measure the partial pressure of carbon dioxide by detecting electromotive force generated by the carbon dioxide. In the present invention, when the system 10 for measuring partial pressure is introduced into the liquid to be measured, the partial pressure is measured by forming a gas-liquid equilibrium state utilizing an extremely small amount of air normally remaining at the holes of the hydrophobic porous membrane 22 and then measuring the partial pressure based on the carbon dioxide having reached the gas-liquid equilibrium state in the air by means of this sensor.

The system 10 for measuring the partial pressure according to this embodiment comprises a hydrophobic porous membrane 22, means for forming the gas-liquid equilibrium state including a carrier gas chamber 34 opening onto the membrane inner surface 32, means for measuring the partial pressure of carbon dioxide including a solid electrolyte sensor 70 disposed on the side of the inner membrane surface 32 and a ceramic heater 72 for heating and thus actuating this sensor, and means for circulating and thus cooling the carrier gas inside the carrier gas chamber 34.

The hydrophobic porous membrane 22 is similar to that in the first and second embodiments, so that a gas-liquid equilibrium state is formed between the liquid in which carbon dioxide is dissolved and the carrier gas inside the carrier gas chamber via the free liquid surface present at each of hole of the porous membrane.

The solid electrolyte sensor 70 must be of a type suitable for the kind of gas to be measured. In this embodiment, a sensor utilizing $Na_3Zr_2Si_2PO_2$ as solid electrolyte and $Li_2CO_3$-$BaCO_3$ as carbonate electrode material was adopted because such a solid electrolyte sensor can detect carbon dioxide but is unaffected by water vapor. The method for manufacturing the solid sensor is as follows.

(1)Firstly, $Zr(OC_2H_5)_4$, $Si(OC_2H_5)_4$, $NaC_2H_5PO(OC_4H_3)_4$ are sequentially dissolved in $C_2H_5OH$.
(2) After they are uniformly dissolved, a large amount of water is added to form hydroxide, and the resultant is then calcined at about 850 °C in an electrical reactor.
(3) The resultant powder is formed into a disk shape by a press, and is then heated to maintained at 1100 °C for twelve hours. This completes the synthesis of the solid electrolyte $Na_3Zr_2Si_2PO_{12}$.
(4) Then, gold paste is baked on to the resultant at between 700 °C and 900°C to form an electrode. Carbonate ($Li_2CO_3$-$BaCO_3$ )is baked there on at 900 °C.
(5) Finally, this electrode is attached to a ceramic heater to complete the formation of the sensor for carbon dioxide.

The ceramic heater 72 is of the conventional type. It is provided in the case of the solid electrolyte sensor because unlike an NDIR the sensor cannot operate unless heated to about 500 °C.

The unit for circulating and thus cooling the carrier gas com prises an air pump 76, a Peltier element 78 and a circulation Tubing 80 for connecting the carrier gas chamber 34, the air pump 76 and the Peltier element 78 to form a circulating passage. This circulating and cooling unit prevents the sea water contacting the porous membrane from being heated by radiant heat from the solid electrolyte sensor 70, which is heated to about 500°C by the ceramic heater 72. Namely, the carrier gas drawn from the carrier gas chamber 34 and heated by the air pump is cooled to a prescribed temperature by the Peltier element 78 and then returned to the carrier gas chamber 34. The Peltier element 78 is well known as means for healing or cooling a liquid passing therethrough by an electric current.

In addition, in order to prevent heating of the liquid, layers of aluminum foil 74 are provided between the hydrophobic porous membrane 22 and the solid electrolyte sensor 70 as a thermal shielding. By appropriately setting the thickness, the area and the number of layers of the aluminum foil 74, heating of the liquid can be prevented to an extent that the measurement value is not affected.

According to the system 10 for measuring partial pressure, a gas-liquid equilibrium state can be formed between the extremely small amount of air remaining at each of the holes and the liquid via the free liquid surface of the liquid presenting at each hole. The solid electrolyte sensor 70 is put in a stand-by state by heating it to a prescribed temperature by the ceramic heater 72. The partial pressure of carbon dioxide can then be measured at a response rate higher than that by the NDIR system by using solid sensor 70 to detect the electromotive force generated in the air by carbon dioxide. Another advantage of the system, according to the embodiment is its high compactness. Still another is that, unlike the NDIR system, it does not require calibration every time measurement is conducted.

Preferred embodiments of the invention have been described with reference to the accompanying drawings, but it is to be understood that the invention is not limited to these precise embodiments, and that various changes and modifications may be effected therein by one skilled in the art without departing from the scope or spirit of the invention as defined in the appended claims. For instance, the subject to be measured is not limited to carbon dioxide dissolved in sea water. Any material dissolved in a liquid can be measured so long as it can be vaporized on the side of the gaseous phase. The NDIR system can, apart from carbon dioxide, also measure carbon monoxide, nitrogen dioxide, nitrogen monoxide, sulfur dioxide, methane and oxygen. The solid electrolyte sensor can measure oxygen, iodine, chlorine, bromine, sulfur dioxide, sulfur trioxide, nitrogen dioxide, hydrogen and sulfur, in addition to carbon dioxide.

The present invention therefore has high potential for application to the measurement of the carbon dioxide, sulfur dioxide, chlorine and other substances with high water solubility.

The depth to which the equilibrator is introduced into the water is a critical factor affecting the hole diameter that can prevent passage of the liquid water due to its surface tension. More specifically, in the case of sea water, for instance, it is estimated that the water pressure is about 1kg/cm$^2$ at most and the surface tension is about $4.0 \times 10^{-2}$ N/m at most. Accordingly, the required diameter is calculated to be 0.1μm to 0.2μm. Since this diameter enables almost all the molecules dissolved in water to pass through the holes, the diameter of the holes does not have to be separately selected every time a different substance is to be measured. In other words, the hydrophobic porous membrane can be widely used.

In addition, although the hydrophobic porous membrane is provided on only one side of the equilibrator in the above embodiments, it can instead be provided on both sides.

As described above, the method and the system for measuring partial pressure of a material dissolved in liquid of this invention enable the partial pressure of the material dissolved in the liquid to be measured with improved practicality and enhanced measurement reliability.

In addition, unlike the conventional bubble and shower systems, when the partial pressure of carbon dioxide dissolved in sea water is continuously measured on the order of ppm level, a direct measurement in sea water can be realized, so that the response can be enhanced and the practicality improved.

**Claims**

1.    A method for measuring partial pressure of a material in a liquid including a step of forming a gas-liquid equilibrium state between the liquid in which the material is dissolved and a carrier gas, and a step of measuring the partial pressure of the material dissolved in the liquid by utilizing vaporized material in the carrier gas which has reached the gas-liquid equilibrium state, characterized in that

the forming step includes a step of forming the gas-liquid equilibrium state between the liquid and the carrier gas present within a certain volume through a free liquid surface existing at each hole of a hydrophobic porous membrane including holes, the diameter and total area of the holes being such that the liquid cannot permeate through the hydrophobic porous membrane due to its surface tension but the material can permeate therethrough.

2.    A method according to claim 1, wherein the material dissolved in the liquid consists of carbon dioxide in sea water, and the gas-liquid equilibrium state is formed by directly inserting the hydrophobic porous membrane into sea water.

3.    A system for measuring partial pressure of carbon dioxide dissolved in a liquid including means for forming a gas-liquid equilibrium state of carbon dioxide between the liquid in which carbon dioxide is dissolved and a carrier gas, and means for measuring the partial pressure of carbon dioxide dissolved in the carrier gas which has reached the gas-liquid equilibrium state, characterized in that

said forming means includes a hydrophobic porous membrane having holes, the diameter and total area of the holes being such that the liquid cannot permeate through the hydrophobic porous membrane due to its surface tension but carbon dioxide can permeate therethrough,
said membrane includes an inner membrane surface contacting the carrier gas and an outer membrane surface contacting the liquid, and
said forming means further includes a carrier gas chamber open onto the inner membrane surface including an inlet opening through which the carrier gas flows in and an outlet opening through which the carrier gas flows out,
whereby a gas-liquid equilibrium state is formed between the carrier gas inside the carrier gas chamber and the liquid through the free liquid surface existing at each hole.

4.    A system according to claim 3, wherein said system further includes means for pumping the liquid toward said outer membrane surface to renew a laminar film on the side of the liquid phase adjacent to said outer membrane surface of said hydrophobic porous membrane.

5.    A system according to claim 4, wherein said hydrophobic porous membrane is coated with a hydrophobic material.

6. A system according to any of claims 3 to 5, wherein said forming means further includes means disposed on said inner membrane surface inside said carrier gas chamber for supporting said hydrophobic porous membrane in the direction of said outer membrane surface.

7. A system according to claim 6, wherein said supporting means includes a plurality of beads of predetermined diameter and the plurality of beads are disposed to contact each other over the entire inner membrane surface, whereby a flow passage for the carrier gas is formed from said inlet opening to said outlet opening of the carrier gas chamber through gaps between the adjacent beads.

8. A system according to claim 7, wherein said outlet opening is disposed remote from said inlet opening and a labyrinth is formed by said carrier gas passage.

9. A system according to claim 7, wherein the system further includes multiple partitions around said outlet opening to guide the carrier gas from said inlet opening to said outlet opening, each of partition including an opening located so that the openings of adjacent partitions are diametrically opposite from each other, whereby said labyrinth is formed by said carrier gas flow passage defined between the adjacent partitions.

10. A system according to claim 7, wherein said system further includes a spiral partition extending from said inlet opening to said outlet opening to guide the carrier gas, whereby said labyrinth is formed by a serpentine carrier gas flow passage defined along said spiral partition.

11. A system according to any of claims 3 to 10, wherein said measuring means consists of a non-dispsersive infrared gas analyzer including a box cell through which sample gas having reached gas-liquid equilibrium state flows, a light source for introducing light into the box cell, and a photodetector for detecting attenuation of the intensity of introduced light owing to absorption by carbon dioxide in the sample gas.

12. A system according to claim 11, wherein said measuring means includes a circulation passage between the box cell and the carrier gas chamber and a circulating pump for circulating the carrier gas via the circulation passage.

13. A system according to claims 11 or 12, wherein a fan for mixing the carrier gas inside the carrier gas chamber is provided inside said carrier gas chamber.

14. A system according to claim 11, wherein said hydrophobic porous membrane is disposed to cover an opening provided on a side of said box cell, whereby a gas-liquid equilibrium state is formed inside the box cell.

15. A system for measuring partial pressure of carbon dioxide dissolved in a liquid including means for forming a gas-liquid equilibrium state of carbon dioxide between the liquid in which carbon dioxide is dissolved and a carrier gas, and means for measuring the partial pressure of carbon dioxide dissolved in the carrier gas which has reached the gas-liquid equilibrium state, characterized in that

said forming means includes a hydrophobic porous membrane having holes of prescribed diameter and total area,
said membrane includes an inner membrane surface contacting the carrier gas and an outer membrane surface contacting the liquid,
a gas-liquid equilibrium state being formed between the liquid and air remaining in the individual holes via the free liquid surface existing at each hole and,
said measuring means includes a solid electrolyte sensor disposed on the side of the inner membrane surface for measuring partial pressure of carbon dioxide by detecting an electromotive force developed by carbon dioxide in the air.

16. A system according to claim 15, wherein said system further includes a thermal shield plate disposed between said hydrophobic porous membrane and said solid electrolyte sensor.

FIG.1

# FIG. 2

# FIG. 3A

# FIG. 3B

# FIG. 4

## TYPE3 + TYPE A

EP 1 043 585 A2

FIG.5

TYPE3 + TYPE B

# FIG.6

TYPE3 + TYPE C

# FIG.7

TYPE2 + TYPE C

FIG.8

TYPE1 + TYPE C

# FIG. 9

# FIG. 10

# FIG. 11

# FIG. 12

# FIG. 13